# EUROPEAN PATENT SPECIFICATION

(11) **EP 1 978 364 B1**
(45) Date of publication and mention of the grant of the patent: **01.06.2011**
(21) Application number: 07007262.4
(22) Date of filing: 06.04.2007
(51) Int. Cl.: G01N 33/564

(54) **Transglutaminase 6 as a diagnostic indicator of autoimmune diseases**
Transglutaminase 6 als diagnostischer Indikator für Autoimmunerkrankungen
Transglutaminase 6 en tant qu'indicateur de diagnostic de maladies auto-immunes

(43) Date of publication of application: 08.10.2008
(73) Proprietor: Zedira GmbH, 64293 Darmstadt (DE)
(72) Inventor: Aeschlimann, Daniel, Dinas Powys CF64 4AB (GB); Hadjivassilliou, Marios, Sheffield S7 1NB (GB)
(74) Representative: Arth, Hans-Lothar

(56) References cited:
- WO-A-01/01133
- WO-A-02/22830
- WO-A2-02/068616
- SBLATTERO DANIELE ET AL: "The analysis of the fine specificity of celiac disease antibodies using tissue transglutaminase fragments." EUROPEAN JOURNAL OF BIOCHEMISTRY, vol. 269, no. 21, November 2002 (2002-11), pages 5175-5181, ISSN: 0014-2956
- PELTOLA M. ET AL: 'Hippocampal sclerosis in refractory temporal lobe epilepsy is associated with gluten sensitivity' JOURNAL OF NEUROLOGY NEUROSURGERY & PSYCHIATRY vol. 80, no. 6, June 2009, pages 626 - 630 ISSN: 0022-3050
- JORUNN STAMNAES ET AL: 'Gliadin T cell epitope targeting by TG3 and TG6: implications for gluten ataxia and dermatitis herpetiformis' PROC. 13TH INT. SYMP. COELIAC DISEASE 01 January 2009, page P-163

## Description

The present invention relates to the diagnosis or to monitoring the effectiveness of therapy of disorders or dysfunctions characterised by autoimmune responses to a novel antigen, transglutaminase 6, by the detection of autoantibodies to the novel antigen.

### Background of the invention

Transglutaminases are a family of structurally and functionally related enzymes that post-translationally modify proteins by catalyzing a Ca²⁺-dependent transferase reaction between the γ-carboxamide group of a peptide-bound glutamine residue and various primary amines. Most commonly, intra- or intermolecular γ-glutamyl-ε-lysine crosslinks are formed by reaction with the ε-amino group of a lysine residue. The action of these enzymes consequently results in the formation of covalently crosslinked, often insoluble supramolecular structures and has a well established role in tissue homeostasis in many biological systems. Nine different transglutaminase genes have been characterised in higher vertebrates on the basis of their primary structure. The respective gene products can be found throughout the body; however, each enzyme isoform is characterised by its own unique tissue distribution, whereby each may be present in a number of different tissues, often in combination with other transglutaminase isoforms.

However, in the absence of suitable amines for crosslinking, transglutaminases hydrolyze peptide-bound glutamine to glutamate (by reaction with H₂O), the biological significance of which has only recently been established in connection with coeliac disease. Coeliac disease is a gluten sensitivity enteropathy that is linked to the consumption of certain cereal proteins called prolamines. Wheat protein, called gluten, contains the prolamine gliadin, which serves as a model for scientific research on coeliac disease. Coeliac disease is a T-cell-mediated autoimmune disorder characterized by its close linkage to specific human lymphocyte antigen alleles: HLA-DQ2 and HLA-DQ8. In susceptible individuals, consumption of gluten triggers a CD4⁺ T-cell response to gliadin as well as a B-cell response to gliadin and self antigens. Transglutaminase 2 (TG2) has been identified as the autoantigen that is recognized in the endomysium of the gut by sera from coeliac disease patients. Activation of gliadin specific CD4+ T-cells by DQ2+ Cd11c+ dendritic cells in the small intestinal mucosa produces proinflammatory cytokines which trigger the organ specific inflammatory reaction. Hallmark symptoms may include weight loss, abdominal bloating and pain and diarrhoea as a consequence of the mucosal lymphocyte infiltration-triggered inflammation in the small bowel which may ultimately cause complete villous atrophy. Characteristically, the condition improves upon gluten exclusion from the diet.

It has previously been demonstrated that TG2 can be used to diagnose autoimmune disorders including coeliac disease and sprue. US 2002/0076834 discloses the diagnosis of celiac disease and sprue by the detection of autoantibodies to TG2. WO 01/01133 discloses the diagnosis of autoimmune disease of the gluten sensitive enteropathy type or of autoimmune diseases associated with gluten sensitive enteropathy by detection of autoantibodies to TG2.

Recently, the present inventors identified a novel transglutaminase gene, TGM6, which encodes the enzyme transglutaminase 6 (TG6) (WO 02/22830) and it was shown to constitute a functional transamidase. RT-PCR analysis of a large number of different human cell lines and tissues revealed that TG6 was identified only in a lung small cell carcinoma cell line (H69).

The present invention surprisingly found that the use of TG6 or an antigenically active fragment thereof, for detection of autoantibodies reacting with transglutaminase 6 is useful for the diagnosis of an autoimmune disorder selected from celiac disease, stiff-man syndrome, gluten sensitive cerebellar ataxia, gluten sensitive peripheral neuropathy, gluten ataxia with enteropathy, headache with white matter abnormality, gluten sensitive myopathy or gluten sensitive ataxia with myoclonus. Thus it is the object of the present invention to provide a method of diagnosis for celiac disease,stiff-man syndrome, gluten sensitive cerebellar ataxia, gluten sensitive peripheral neuropathy, gluten ataxia with enteropathy, headache with white matter abnormality, gluten sensitive myopathy and gluten sensitive ataxia with myoclonus.

The object of the present invention is solved by the teaching of the independent claims. Further advantageous features, aspects and details of the invention are evident from the dependent claims, the description, and the examples of the present application.

### Description of the invention

The transglutaminase isoforms TG5, TG6 and TG7 have been cloned by us (WO 02/22830) and as demonstrated in Example 1 of the present application one of these, TG6, is predominantly expressed by a subset of neurons in the central nervous system including Purkinje cells. The preferential expression of TG6 in neural tissue provides a clear possibility that this enzyme could be involved in the pathogenesis of autoimmune disorders, and particularly in the pathogenesis of autoimmune disorders with neurological symptoms.

The present invention provides a method to detect autoantibodies in suitable body fluids by means of an immune reaction with a novel antigen, transglutaminase 6 (TG6). The antigen may adopt the form of native or denatured protein, or fragments or peptides thereof, or may be presented in the form of mixtures or extracts containing TG6 or parts thereof. The method provided may be used for the diagnosis of diseases, or the monitoring and assessment of the effectiveness of therapy, especially autoimmune diseases and in particular autoimmune diseases with neurological symptoms, preferably but not exclusively of the cereal protein sensitive type including ataxia and neuropathy.

As used in this application the term transglutaminase 6 includes human or animal transglutaminase 6 and also includes naturally produced or recombinantly produced transglutaminase 6.

Thus the present invention is directed to the use of transglutaminase 6, or an antigenically active fragment thereof, for the detection of autoantibodies reacting with transglutaminase 6 for the diagnosis of an autoimmune disorder,

wherein the disorder is gluten sensitive cerebellar ataxia, gluten sensitive peripheral neuropathy, gluten sensitive myopathy, gluten sensitive ataxia with myoclonus, celiac disease, gluten ataxia with enteropathy,

headache with white matter abnormalities, or stiff-man syndrome.

Preferably the autoantibodies are detected in a patient body fluid sample. Preferably the patient body fluid sample is a sera sample. Further preferably the autoantibodies are detected in a patient tissue sample.

Preferably transglutaminase 6 used to detect the autoantibodies is purified from human tissue. Further preferably the transglutaminase 6 used to detect the autoantibodies is purified from animal tissue. Further preferably the transglutaminase 6 is produced by recombinant DNA technology, based on either the human or an animal or a mixed gene sequence.

Preferably the antigenically active fragment of transglutaminase 6 is a synthetic peptide.

In practice the transglutaminase 6 used to detect autoantibodies can be comprised of a mixture of any of a transglutaminase 6 purified from human tissue, a transglutaminase 6 purified from animal tissue, a transglutaminase 6 produced by recombinant DNA technology and a synthetic peptide of an antigenically active fragment of transglutaminase 6.

In the present invention the detection of transglutaminase 6 autoantibodies can be combined with the detection of autoantibodies to one or more further transglutaminase isoforms. The one or more further transglutaminase isoforms can be selected from transglutaminase 1, transglutaminase 2, transglutaminase 3, transglutaminase 4, transglutaminase 5, transglutaminase 7, coagulation factor XIII or erythrocyte membrane protein band 4.2.

### Description of the Figures

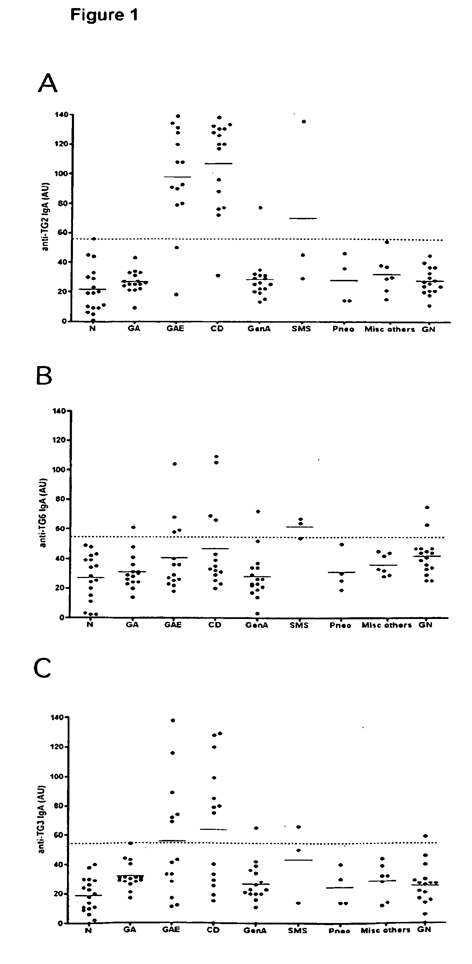

Figure 1. Analysis of serum anti-TG2 IgA (A), anti-TG6 IgA (B), anti-TG3 IgA (C). N, healthy blood donor; GA, gluten ataxia; GAE, gluten ataxia with enteropathy; CD, untreated celiac disease; GenA, genetic ataxia; SMS, stiff man syndrome; Pneo, paraneoplastic syndromes; Misc others, unrelated neurological condition; GN, gluten sensitive peripheral neuropathy.

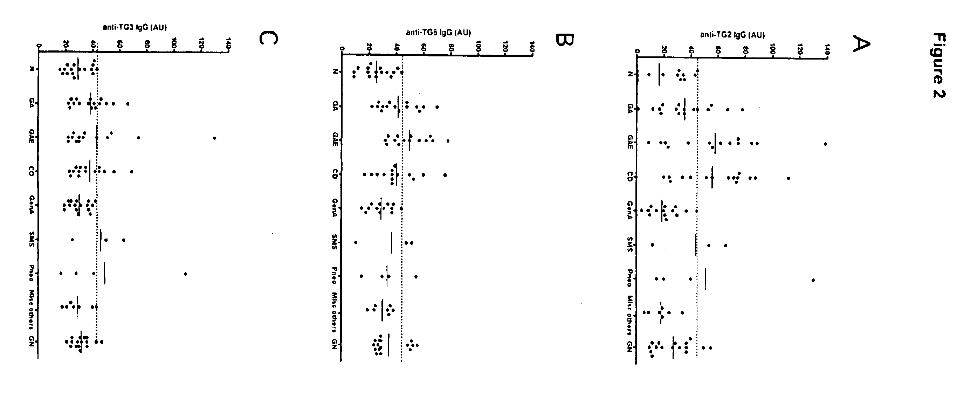

Figure 2. Analysis of serum anti-TG2 lgG (A), anti-TG6 lgG (B), anti-TG3 lgG. Note, 1 GAE patient is displayed as the maximum (140AU) but the reading exceeded the range (227AU). N, healthy blood donor; GA, gluten ataxia; GAE, gluten ataxia with enteropathy; CD, untreated celiac disease; GenA, genetic ataxia; SMS, stiff man syndrome; Pneo, paraneoplastic syndromes; Misc others, unrelated neurological condition; GN, gluten sensitive peripheral neuropathy.

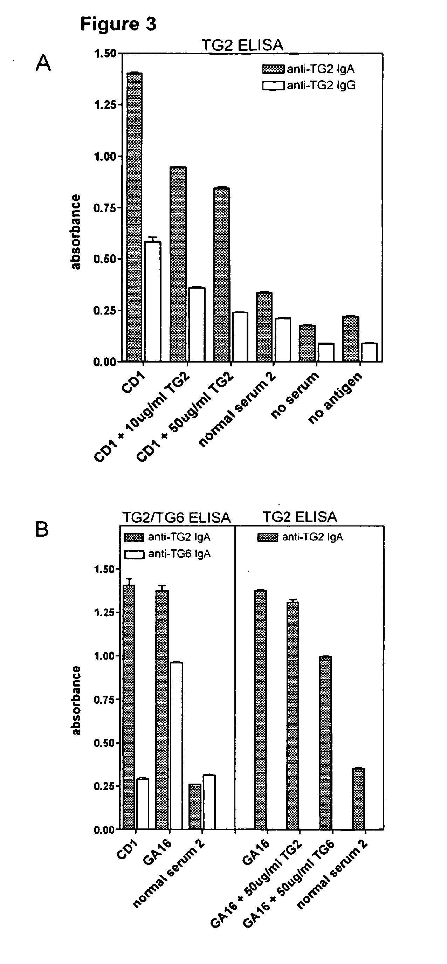

Figure 3. Effect of preincubation of sera with selected antigens on the antibody reaction measured by ELISA. *A,* TG2 IgA and lgG ELISA results for an example of a serum sample from a blood donor (normal serum 2) and from a celiac disease patient (CD1) with or without incubation with 10 or 50 µg/ml TG2 and relevant controls. *B*, TG2 lgA and TG6 lgA ELISA results for a serum sample of the blood donor (normal serum 2), celiac disease patient (CD1) and a gluten ataxia patient (GA16) (left panel) and TG2 lgA ELISA results for the same ataxia patient serum after preincubation with either TG2 or TG6.

### EXAMPLES

Whilst the role of transglutaminases in degenerative neurological diseases has been intensely investigated, their role in the development of immune mediated neurological dysfunction other than gluten sensitivity has not been evaluated. Thus in addition to the neurological manifestations of gluten sensitivity we explored transglutaminase-linked autoimmune responses in patients with paraneoplastic neurological syndromes and patients with stiff man syndrome.

### Example 1: Expression of TG6 isoform in neurons of central nervous system

Cloning of mouse TG6 - Mouse brain (BALB/c strain) was dissected, rinsed in PBS and immediately frozen on dry ice. The frozen brain was homogenised in 1 ml of Tri Reagent (Sigma) using a teflon pestle and total RNA was prepared by chloroform extraction and isopropanol precipitation following the manufacturer's instructions. cDNA was synthesised from 2µg total RNA by reverse transcription using 200 units of SuperScript II RNase H⁻ Reverse Transcriptase (Invitrogen) and 5µM oligo(dT)₁₅ primer in a total volume of 20µl. For the cloning of mouse TG6, a series of gene-specific oligonucleotides modelled from the human TG6 sequence (WO 02/22830) were used to isolate overlapping DNA fragments by PCR. The full-length nucleotide sequence of mouse TG6 was deduced and deposited into the GenBankTM/EBI Data Bank under accession number AY159126.

In situ hybridization - A 325bp fragment corresponding to the 3' end of mouse TG6 was generated by PCR. The fragment was cloned into the pCRII vector using TA cloning (Invitrogen). For *in vitro* transcription, the cDNA fragment with flanking RNA polymerase promoters was excised by restriction with *Pvu*ll and *Afl*III and isolated using the QlAquick Gel extraction kit (Qiagen). Digoxigenin-UTP (DIG)-labelled, single-strand antisense and sense RNA probes were prepared using the DIG RNA Labelling Kit (Roche) with 1µg purified DNA fragment and 40 units of either RNA Polymerase SP6 or T7 in 20µl transcription buffer supplemented with 0.1 mM ATP, 0.1 mM CTP, 0.1 mM GTP, 0.065mM UTP, and 0.035mM DIG-11-UTP following the manufacturer's instructions. DNA template was degraded by incubation with 20 units RNase-free DNase I (Roche) for 15 min at 37°C, the reaction terminated by addition of 0.2M EDTA (pH 8.0) to a final concentration of 0.02M, and after supplementation with 0.4M LiCl, the labelled RNA collected by ethanol precipitation. To determine the yield of labelled RNA, the RNA was compared to a dilution series of a DIG-labelled control RNA by spotting onto a Nytran nylon membrane (Schleicher and Schuell) and visualisation using the DIG Nucleic Acid Detection kit (Roche).

Newborn mouse and mouse embryos at gestation days 11, 13 and 16 were fixed in 4% paraformaldehyde in PBS, pH 7.4, at 4°C overnight. To improve penetration of fixative into the newborn mouse, an excision was made along the abdomen with a scalpel. The mice were transferred to 0.5% paraformaldehyde in PBS containing 0.42M EDTA for 4 days for demineralisation of skeletal tissues, washed in PBS, then processed through a graded ethanol series and paraffin embedded. Sagittal sections of 5µM thickness were cut, transferred onto gelatin coated glass slides and dried overnight at 50°C. Following deparaffinisation, tissue sections were washed with DEPC treated H₂O treated with proteinase K (Sigma; 4µg/ml in 100mM Tris-HCl, pH 8, 50mM EDTA) at 4°C for 15 min before being acetylated with 0.25%(v/v) acetic anhydride in 0.1 M triethanolamine, pH 8.0 for 10 min. Sections were prehybridised for 15 min at 37°C in 5xSSC, followed by hybridisation overnight at 42°C with 20ng/µl of the DIG labelled probe in 30µl hybridisation solution consisting of 50% formamide, 10% dextran sulphate, 5xSSC and 300µg/ml herring sperm DNA (Sigma) in DEPC treated H₂O (Frame-Seal incubation chambers [MJ Research, Inc.] were used to eliminate evaporation of reagents during hybridisation). Slides were subsequently washed in 2xSSC for 30 min at room temperature, twice in 2xSSC for 20 min at 37°C, and once in 1xSSC for 20 min at 37°C. Hybridised probe was subsequently visualised using the DIG nucleic acid detection kit (Roche) according to the manufacturer's instructions by incubation with alkaline phosphatase-labelled anti-DIG antibody (diluted 1:500) and subsequent development with NBT/BCIP substrate solution for 12 hours.

Isolation of cortical neurons - Cerebral cortex of newborn Balb C mice was dissected on ice in Hanks balanced salt solution (HBSS) and digested for 20 min at 37°C in 0.1% trypsin, 0.05% DNAse I (Sigma) in HBSS. The tissue was washed and subsequently triturated in HBSS/DNAse I solution to dissociate cells. Cells were washed and subsequently maintained in DMEM/F12 containing 2% B27 supplement (Invitrogen) and for culture, also 20ng/ml bFGF, 20ng/ml EGF, 100 units/ml penicillin G and 100µg/ml streptomycin. Number of vital cells was determined by trypan blue exclusion and for immunocytochemistry, cells seeded at a density of 1x10⁵ cells/well in 12-well plate on laminin-1 coated cover slips and grown for 5 days (37°C/5%CO₂). For FACS analysis, cells were washed in 0.5% BSA in phosphate-buffered saline, pH 7.4 (PBS), filtered through 40µm Falcon cell strainers (Becton Dickinson) to remove remaining aggregates and fixed for 20 min on ice in 2% paraformaldehyde in PBS.

FACS analysis - Cells were permeabilized in PBS containing 0.5% saponin for 20 min on ice. After three washes in PBS containing 0.1 % saponin, non-specific binding was blocked in TBS containing 1% BSA and 3mg/ml rabbit anti-mouse IgG (DAKO) and then cells incubated with monoclonal antibodies against glial fibrillary acidic protein (G-A-5, Sigma, 15µg/ml), β-tubulin isoform III bodies (SDL.3D10, Sigma, 20µg/ml), oligodendrocytes (RIP, Chemicon,1:1000 diluted) and goat anti TG6 antibodies (20µg/ml) in TBS/BSA overnight at 4°C. Primary antibody binding was detected by incubation with 13µg/ml FITC-conjugated rabbit anti mouse (MP Biomedicals) and R-phycoerythrin rabbit anti goat (Sigma) secondary antibodies for 60 min at room temperature. Analysis was performed immediately after labelling using a FACScalibur flow cytometer (Becton Dickinson) equipped with an argon laser emission wavelength of 488nm. FITC and PE signals were identified using 530 and 585 band pass filters, respectively. The analysis was performed using Cell Quest software (Becton Dickinson). Ten thousand events were acquired for each sample. Background level of fluorescence was determined from controls with non-specific IgG (ChromPure goat/mouse IgG, Jackson ImmunoResearch Labs Inc) replacing the primary antibodies.

### Results:

We previously cloned human TG6 from a carcinoma cell line (WO 02/22830). To obtain a clearer understanding of TG6 expression on a cellular level, *in situ* hybridisation was performed on sagittal newborn mouse sections. A 325bp fragment corresponding to the 3' end of TG6 was used as a probe as this area has the least homology between the different TGs and a similar human probe gave no cross-hybridisation with other TG gene products in Northern blotting. *In situ* hybridisation revealed that TG6 is expressed in the brain, within the cell layers containing the neuronal cell bodies of the cerebral cortex (particularly layers II-IV containing granular neurons and pyramidal cells), and the cerebellum (Purkinje cells). TGase 6 was also expressed in neurons of the spinal cord and the retinal cells of the eye.

After identifying that TG6 is expressed predominantly in the central nervous system in the developed organism, we were interested to identify whether the induction of TG6 expression correlated with any specific events in development. While the central nervous system is the first organ system to develop and to differentiate, it is also one of the last to be completed. Simplistically however, the primary parts of the brain can be identified soon after the neural groove, neural plate and head process stage at embryonic day 7.5, and by embryonic day 14 the brain is typically that of a mammal. *In situ* hybridisation was therefore carried out on mouse embryos at days 11, 13.5 and 16 of gestation (the 11, 13.5 and 16 day mouse embryo is comparable to the 30 day, 38 day, and 10.4-week human embryo, respectively). The walls of the primitive brain divide into an inner ependymal, an intermediate mantel, and outer marginal layer by day 10, whereby the ependymal layer that ultimately forms the lining of the ventricles of the brain is the thickest layer. At embryonic day 11, active proliferation of neuroblastic cells occurs in the walls of the entire central nervous system and these begin to occlude some of the neural cavities. Up to day 11, the major neuroblastic activity is occurring behind the hindbrain where cranial ganglia V to IX develop. Little TG6 expression was detected in the brain at day 11 while extensive labelling was seen in the developing spinal cord. By day 13, TG6 expression was apparent in several parts of the brain and strong staining could be detected in regions undergoing neuronal differentiation such as the mesencephalon. From days 13-16, the major neuroblastic activity occurred in the cerebral cortex (telencephalon) where cells from the mantle layer migrate into the overlying marginal zone to form the neopallial cortex which will become the outer grey matter of the cerebral hemispheres. By day 16, TGase 6 was highly expressed in the cerebral cortex and the expression pattern was comparable to the expression in the fully developed brain. Induction of TG6 expression appears to correlate both spatially and temporally with neurogenesis.

To further characterize the cell population expressing TG6, we used flow cytometry and specific antibodies against intracellular markers to discriminate between different cell types such as neurons, astrocytes or microglial cells. Cells of the neuronal lineage were identified using antibodies against β-tubutin III (Tuj-1), astrocytes with antibodies to glial fibrillary acidic protein (GFAP) and oligodendrocytes with RIP-antibodies, a method verified to be reliable for characterisation of CNS-derived cells. Polyclonal antibodies to TG6 were raised against a synthetic peptide corresponding to the connecting loop between the catalytic core and β-barrel 1 domain of TG6, purified by affinity chromatography on the synthetic peptide and verified to be specific by immunoblotting of recombinant protein and tissue extracts. Cells were isolated from the cerebral cortex of newborn mice and for analysis using FACS, immediately fixed, permeabilized and double-labelled with antibodies to TG6 and to one of the cell markers. Physical parameters were used to distinguish neurons astrocytes and microglial cells as they differ in size and morphology. Therefore forward scatter (FSC), representing cell size, was plotted as a function of fluorescence intensity for TG6 labelling. Within the broad distribution of cells expressing TG6, two clusters of cells of different size were apparent and were gated. Further analysis of gated cells for expression of cell markers showed that both clusters were exclusively positive for β-tubulin III indicating that they are derived from the neuronal lineage and represent different neuronal populations. lmmunohistochemistry confirmed the absence of TG6 from the astroglial and oligodendroglial lineage and expression in a subset of neuronal cells.

### Example 2: Detection of antibodies to TG isoforms in sera of control subjects and patients with celiac disease or unexplained neurological dysfunctions.

Transglutaminase 2, 3 and 6 were produced recombinantly in E. coli and purified by known techniques. The purified proteins gave a single band on Coomassie blue stained SDS-PAGE gels and their identity was verified by demonstrating enzymatic activity, immunoreactivity with the respective antibodies, and peptide fingerprinting and/or sequencing using MALDI-TOF mass spectrometry (MS) and tandem MS.

Sera of patient groups with unexplained neurological dysfunction:16 gluten ataxia-without enteropathy (GA); 14 gluten ataxia with enteropathy (GAE); 16 peripheral neuropathy (GN); 3 stiff man syndrome (SMS) and 4 paraneoplastic syndrome (Pneo) was analysed and compared with sera of various control groups: 16 genetic ataxia (GenA); 16 classical celiac disease (CD) and 18 healthy controls (N).

All patients had been examined at the Gastroenterology and Neurology Departments of the Royal Hallamshire Hospital, Sheffield, UK. Collection and analysis of sera has been approved by the research ethics committee (REC 06/Q2307/6).

The gluten ataxia group was defined as sporadic cerebellar ataxia associated with the presence of antigliadin antibodies and the absence of an alternative etiology for ataxia. This group was split into a subgroup with enteropathy (GAE) and a subgroup without enteropathy (GA). These patients are characterised by uncoordinated movement due to the abnormality of muscle control or an inability to finally coordinate movements, resulting in a jerky, unsteady, to-and-for motion of the trunk or the limbs, due to cerebellar degeneration. A group of patients with ataxia of known genetic origin served as a control.

Peripheral neuropathy is a progressive dysfunction of the nerves that carry information to and from the brain and spinal cord. This produces pain, loss of sensation and inability to control muscles. Gluten sensitive peripheral neuropathy (GN) is prevalent among patients with idiopathic peripheral neuropathy at about 40%.

Stiff man syndrome (SMS) is a rare disorder characterized by severe progressive muscle stiffness of the trunk and lower limbs with painful spasms. An autoimmune aetiology is suggested by the association with HLA DR3 or DR4, the presence of serum anti-GAD (glutamic acid decarboxylase) antibodies, and the benefit from intravenous immunoglobulin therapy.

The paraneoplastic group (Pneo) included patients with neurological dysfunction as a consequence of various malignancies including lymphoma or small cell carcinoma of lung or ovarian origin. Whilst neuronal antibodies that recognise antigens on transformed cells have been well characterised (anti-Hu, anti-Yo), these antibodies have not been found to be pathogenic. It is possible therefore that neural damage in these syndromes has an alternative explanation involving "tissue-type" transglutaminase isoforms. The rationale for this is based on the fact that TG6 has been produced by a cell line derived from small cell carcinoma of the lung.

Sera of patient groups with neurological symptoms were compared to samples from a group of blood donors of unknown history (N) and a group of diagnosed celiac disease patients (CD).

Enzyme Linked Immunosorbent Assay (ELISA): High capacity protein binding 96-well plates (Immulon^{®} 2HB, Thermo Electron) were coated with 100µl/well of 5µg/ml antigen (TG2, TG3 or TG6) in TBS (20mM Tris HCl, pH 7.4, 150mM NaCl overnight at 4°C. All binding steps were followed by 5 rinsing steps with TBS containing 0.01% Tween 20 and all subsequent incubations were carried out at room temperature. Non-specific binding was blocked by incubation with 200µl/well of 3% BSA in TBS for 60min. Patient sera were diluted 1:100 in 1% BSA in TBS and any protein aggregates present removed by centrifugation at 10,000 x g for 5min. Coated plates were incubated with 100µl/well of cleared patient sera for 90 min. After rinsing, serum antibody binding was detected by incubation with 100µl/well of either peroxidase-conjugated affinity pure goat antihuman lgA (Jackson Immuno Research; diluted 1: 2000 in 1% BSA/TBS) or rabbit antihuman IgG (Dako; diluted 1: 1000 in 1% BSA/TBS) for 90 min. The reaction was finally developed for 30 min using 5-amino-2-hydroxybenzoicacid/NaOH, pH 6.0, 0.005% H₂O₂, as a peroxidase substrate solution (100µl/well) and stopped by addition of 100µl 1 M NaOH to each well. After 15 min, the absorbance at 490nm was measured.

Inhibition ELISAs followed the protocol above but the test sera were incubated with a dilution series of recombinant transglutaminases for 90min at 37°C while shaking prior to addition to the ELISA plate..

The amount of protein and serum dilutions used in the test was optimised. All serum samples were analysed twice in triplicates on wells containing antigen or which were blocked with BSA only. A selected negative and a selected positive reference serum, as well as a buffer blank, were also run in parallel on each plate. The BSA only background was subtracted, and the antibody reading expressed in arbitrary units as a percentage of the reference sera.

Statistics - Based on previous work on coeliac patient sera we know that data from such assays do not show a Gaussian distribution but that the difference between means of positive and negative serum tests is very large. Conservatively assuming that a difference of 50 units will be sufficient to differentiate between the respective groups non-parametric analysis (Mann-Whitney test) indicates that a sample size of 10 per group is sufficient to provide a power of 90% at a significance level of 0.05.

### Results:

Human TG6 and other transglutaminase isoforms were expressed using recombinant DNA technology and ELISAs performed based on the respective purified proteins for detection of lgG and lgA in serum and cerebrospinal fluid (CSF). Recombinant human proteins were chosen over proteins purified from animal tissue as this has been reported to increase the sensitivity of the assay in the case of TG2 (WO 01/0133). ELISAs were performed using the same antigen concentration for coating, serum dilution, and reaction time for assay development. At serum dilutions of 1:100 or less, some negative sera showed increased signal while the signal in some positive sera reached a plateau. For sera from gluten ataxia patients, the signal to background ratio was highest at a dilution of 1:100 and hence, this dilution was used for all assays. A small number of sera produced abnormally high signal on wells incubated with blocking agent but in the absence of antigen. High serum immunoglobulin concentrations have been reported to cause false-positive classification in TG2 lgA assay, therefore all assays were carried out in the presence and absence of antigen and the relative signal was used for analysis. A selected positive and negative reference serum was included in each assay to control for assay performance and for data normalization into arbitrary units as a function of the reference samples. In house assay performance was further evaluated against a commercially available clinical assay for TG2 lgA (Genesis Diagnostics) and the mean interassay variation was found to be 11.0% and 3.6% for CD and healthy groups, respectively. We also investigated whether the results were dependent on the conformational state of the antigen. For this purpose, antigen (TG2 or TG6) was pretreated and coated at 4°C in buffer containing 5mM CaCl₂ (Ca₂-activated form), 1 mM EDTA (reversibly inactivated conformation) or 2M urea (partially denatured form; note, conformational changes induced by urea >1 M are irreversible). For TG2 IgA, conformational changes induced by EDTA or urea reduced the signal in positive serum samples from 0.97±0.05 to 0.8±0.06 and 0.8±0.06, respectively, and in negative serum samples from 0.12±0.01 OD to 0.1±0.01 and 0.08±0.01 OD, respectively. The reduction in signal was identical with either treatment and ranged from 11 to 30% for individual patients. Similar results were obtained for TG6 IgA and IgG sera analysis. These data are consistent with data in the literature (WO 01/01133) and suggest that antigen conformation has no principal effect on the performance of the test although the signal was significantly higher with the Ca₂-activated form of the enzyme for all patients tested. Samples were strictly kept at 4°C for coating and it is therefore unlikely that autocatalytic crosslinking played a major role in the enhanced antibody binding.

The median antibody concentrations (in AU) determined by the ELISAs for TG2, TG6, and TG3 are presented in Figures 1 and 2.

The coincidence of the TG2 lgA assay with the clinical diagnosis of celiac disease was 15/16 (94%) with the remaining individual having antibodies to TG3 only (Figure 1). 7 (47%) of these 15 patients were also positive for TG3 IgA and 5/15 (33%) had lgA to TG6. lgG titres were generally lower and less predictive with only 10/16 (63%) having a positive result for TG2 lgG (Figure 2).

Ataxia of the gluten sensitivity type was characterized by an antibody response to gliadin (Table 1). Gluten ataxia patients were further grouped into two subgroups, those with enteropathy and those without, after it became clear that enteropathy correlated with TG2 IgA (Figure 1). For those with gastrointestinal disease, transglutaminase antibodies were an excellent predictor with 12/14 (86%) having TG2 IgA titres similar to coeliac disease patients and a positive result in the endomysial antibody (EMA) test (Table 1). 50% of these individuals were also positive for TG3 IgA and 33% were positive for TG6 IgA, a pattern identical to that of patients with intestinal manifestation of coeliac disease. This is also consistent with all but 1 (DQ4) of these carrying HLA DQ2 (Table 1). The remaining two individuals of this group (GA19 & GA32) were EMA negative and transglutaminase IgA negative but had lgGs to TG6 and TG2 (Table 1) suggesting that based on gastrointestinal symptoms, these individuals were assigned to the incorrect group. For the gluten ataxia group without enteropathy, none of the16 patients tested positive for EMA or TG2 lgA (Table 1). However, 8/18 (44%) (after reassignment of the above 2 individuals) patients had anti-TG6 IgGs and 12/18 (67%) had anti-transglutaminase lgG of one or more type (Fig 3.2). It is interesting to note that anti-TG6 lgG was also more prevalent in the ataxia/enteropathy group (50%) than in the coeliac disease group (29%). Also, 3 patients with anti-transglutaminase lgG had HLA DQ1 suggesting a distinct molecular mechanism.

The EMA result and the HLA type and gliadin antibody type for the gluten ataxia patients, with and without enteropathy, and for the genetic ataxia patients are summarized in Table 1 below. Also presented in Table 1 is the TG antibody concentration raw data determined by ELISA and used to generate Figures 1 and 2.

**Table 1.**

| Patient | HLA type | EMA | α gliadin | α TG2 IgA | α TG6 IgA | α TG3 IgA | α TG2 IgG | α TG6 IgG | α TG3 IgG |
|---|---|---|---|---|---|---|---|---|---|
| Gluten ataxia | | | | | | | | | |
| GA 2 | DQ1 | negative | IgG | 27±2 | 30±2 | 31±2 | 42±2 | 26±1 | 39±4 |
| GA 3 | DQ1 | negative | IgA | 33±3 | 48±1 | 44±6 | 55±6 | 60±3 | 45±5 |
| GA 4 | DQ1 | negative | IgG | 24±2 | 28±2 | 29±1 | 53±3 | 42±4 | 46±3 |
| GA 7 | DQ2 | unknown | IgG | 21±3 | 24±2 | 31±2 | 29±5 | 57±1 | 38±3 |
| GA 12 | DQ1 | negative | IgG | 43±3 | 26±1 | 21±1 | 31±5 | 22±1 | 42±2 |
| GA 20 | DQ8 | negative | IgG/IgA | 25±3 | 23±2 | 28±1 | 12±7 | 48±2 | 41±1 |
| GA 21 | DQ1 | negative | IgG/IgA | 9±4 | 24±1 | 29±3 | 0±10 | 27±1 | 24±1 |
| GA 22 | DQ2 | negative | IgG/IgA | 25±1 | 31±4 | 28±2 | 78±4 | 70±1 | 66±5 |
| GA 23 | DQ2 | negative | IgG | 31±2 | - | 40±4 | 67±4 | - | 30±2 |
| GA 24 | DQ2 | negative | IgG | 22±1 | 14±2 | 17±2 | 31±2 | 39±2 | 28±1 |
| GA 25 | DQ2 | negative | IgG/IgA | 34±3 | 41±3 | 31±1 | 35±1 | 30±2 | 55±2 |
| GA 26 | DQ2 | negative | IgG/IgA | 25±2 | 36±2 | 26±2 | 18±1 | 48±1 | 22±1 |
| GA 27 | DQ2 | negative | IgA | 26±1 | 31±0 | 30±4 | 45±1 | 35±1 | 26±2 |
| GA 28 | - | negative | lgG | 27±1 | 29±2 | 43±2 | 35±2 | 33±1 | 23±1 |
| GA 29 | DQ2 | negative | lgG | 21±1 | 20±2 | 31±5 | 19±2 | 29±1 | 50±3 |
| GA 30 | DQ1 | negative | lgG | 33±2 | 61±2 | 54±4 | 27±1 | 55±2 | 37±1 |

| Gluten ataxia with enteropathy | | | | | | | | | |
|---|---|---|---|---|---|---|---|---|---|
| GAE 1 | DQ2 | positive | IgG/IgA | 91±4 | 25±1 | 89±4 | 62±5 | 57±1 | 130±0 |
| GAE 5 | DQ2 | positive | IgG/IgA | 108±4 | 27±1 | 33±4 | 75±2 | 62±2 | 51±1 |
| GAE 8 | DQ2 | positive | IgG/IgA | 79±3 | 22±2 | 11±3 | 56±2 | 32±1 | 25±1 |
| GAE 11 | DQ2 DQ8 | positive | IgG/IgA | 108±3 | 40±1 | 72±5 | 69±3 | 50±2 | 54±4 |
| GAE 14 | DQ2 | positive | IgG/IgA | 120±3 | 36±2 | 17±1 | 38±3 | 46±1 | 28±1 |
| GAE 16 | DQ4 | negative | IgA | 93±4 | 104±5 | 138±15 | 9±8 | 42±4 | 22±1 |
| GAE 17 | - | positive | IgG | 80±3 | 23±1 | 43±5 | 23±6 | 41±1 | 33±1 |
| GAE 19 | DQ2 | positive | IgG | 18±3 | 29±2 | 12±4 | 75±3 | 78±1 | 43±3 |
| GAE 31 | DQ2 | positive | IgG/IgA | 142±8 | 53±1 | 41±4 | 18±3 | 51±3 | 26±1 |
| GAE 32 | DQ2 | negative | IgG | 50±2 | 26±1 | 28±5 | 54±3 | 67±1 | 30±1 |
| GAE 33 | DQ2 | positive | IgG | 90±5 | 18±2 | 33±2 | 85±2 | 34±2 | 22±1 |
| GAE 34 | DQ2 | positive | IgG/IgA | 131±7 | 68±0 | 69±10 | 21±1 | 39±1 | 30±2 |
| GAE 35 | DQ2 | positive | IgG/IgA | 134±7 | 58±1 | 116±1 | 89±4 | 33±1 | 74±2 |
| GAE 36 | DQ2 | positive | IgG | 128±4 | 36±1 | 74±1 | 212±5 | 65±1 | 34±4 |

| Genetic ataxia | | | | | | | | | |
|---|---|---|---|---|---|---|---|---|---|
| GenA 1 | DQ3 | negative | negative | 22±3 | 23±1 | 28±4 | 11±9 | 37±1 | 23±2 |
| GenA 2 | - | negative | negative | 29±1 | 34±1 | 23±3 | 30±6 | 44±1 | 37±2 |
| GenA 3 | DQ2 | negative | negative | 15±3 | 14±2 | 11±4 | 0±8 | 15±1 | 19±2 |
| GenA 4 | DQ1 | negative | negative | 19±1 | 17±1 | 20±3 | 20±7 | 31±1 | 28±2 |
| GenA 5 | DQ1 | negative | negative | 32±2 | 37±3 | 36±2 | 37±9 | 37±1 | 28±1 |
| GenA 6 | - | negative | negative | 31±1 | 52±0 | 42±3 | 45±3 | - | 38±3 |
| GenA 7 | DQ1 | negative | negative | 25±4 | 34±1 | 39±3 | 0±9 | 22±1 | 24±2 |
| GenA 8 | DQ2 | negative | negative | 20±3 | 21±2 | 16±4 | 9±6 | - | 37±2 |
| GenA 9 | DQ2 | negative | negative | 77±2 | 72±1 | 65±3 | 22±7 | 28±2 | 42±3 |
| GenA 10 | DQ1 | negative | negative | 13±2 | 3±3 | 0±3 | 15±6 | 28±2 | 31±2 |
| GenA 11 | DQ1 | negative | negative | 26±4 | 23±2 | 21±3 | 29±6 | 34±1 | 40±2 |
| GenA 12 | DQ2 | negative | negative | 25±3 | 22±2 | 20±2 | 21±7 | 38±1 | 29±1 |
| GenA 13 | DQ2 | negative | negative | 29±2 | 19±2 | 26±4 | 10±3 | 18±1 | 19±2 |
| GenA 14 | DQ8 | negative | negative | 35±1 | 26±1 | 20±3 | 4±4 | 20±1 | 22±2 |
| GenA 15 | DQ2 | negative | negative | 22±1 | 26±2 | 23±1 | 27±4 | 26±1 | 27±2 |
| GenA 16 | DQ3 | negative | b IgA | 31±1 | 29±2 | 34±7 | 21±2 | - | 36±2 |

Antibodies to TG6 were also prevalent in patients with the rare neurological condition stiff man syndrome, with 3/3 having anti-TG6 IgA and 2/3 anti-TG6 lgG. While one of these had coeliac disease (EMA positive and high TG2 lgA titre), the other 2 had a prevalent lgG response.

In the control groups (16 blood donors and 16 genetic ataxia) only 1 patient had any antibodies to transglutaminase isoforms. This patient had the risk HLA DQ2, moderate titres of lgA to TG2, TG3 and TG6 and is likely to be a clinically silent celiac disease patient. Overall, anti-TG6 IgG was more frequent than any other anti-transglutaminase antibodies in patients with neurological dysfunctions.

To assess whether isoform-specific antibodies were present or the same antibodies cross-reacted between enzyme isoforms, inhibition studies were carried out. Using coeliac disease sera with reaction to TG2 only (Figure 3B, left panel), we could show a dose-response whereby the highest concentration employed, 50µg/ml of TG2, completely (93%) blocked IgG detection and partially (52%) blocked IgA detection (Fig 3.3A). On the other hand, TG6 was much more effective (37%) than TG2 (6%) in blocking the signal produced by gluten ataxia sera which tested positive for lgA to TG6 and other transglutaminase isoforms (Figure 3B). This result together with the finding that a number of patients exclusively tested positive for anti-TG6 lgG (Table 1) provides evidence that some patients develop populations of antibodies which are specific for or have greater avidity for TG6 than other enzyme isoforms as has been observed for lgA to TG3 in dermatitis herpetiformis patients. Our results demonstrate that a subgroup of patients with neurological dysfunction due to autoimmune processes develop a TG6-specific B-cell response.

We have shown that antibodies against TG6 can serve as a marker in addition to HLA type, EMA test, and detection of antigliadin and anti-TG2 antibodies to identify a subgroup of patients with gluten sensitivity. While anti-TG IgA response is linked with gastrointestinal disease, an anti-TG IgG response is prevalent in gluten sensitive ataxia independent of intestinal involvement but such a response is absent in ataxia of defined genetic origin or healthy individuals. Consequently, we provide a marker that aids the identification of patients with autoimmune-based neurological dysfunction. A number of methods are commonly used for the diagnostic detection of antibodies in samples of body fluids; examples for such methods include: ElA/ELISA, LiA, FiA, RIA, IRMA, IEMA/EIA, ILMA, IFMA, immunodiffusion, Western-blot or Dot-blot. Any of these methods could be adapted for diagnostic purposes following the method described herein in detail by a person skilled in the art.

## Claims

1. Use of transglutaminase 6, or an antigenically active fragment thereof, for the detection of autoantibodies reacting with transglutaminase 6 for the *in-vitro* diagnosis of an autoimmune disorder or for monitoring the effectiveness of therapy of an autoimmune disorder, wherein the autoimmune disorder is celiac disease, stiff-man syndrome, gluten sensitive cerebellar ataxia, gluten sensitive peripheral neuropathy, gluten ataxia with enteropathy, headache with white matter abnormality, gluten sensitive myopathy or gluten sensitive ataxia with myoclonus.

2. Use according to claim 1, wherein the detection of transglutaminase 6 autoantibodies is combined with the detection of autoantibodies to one or more further transglutaminase isoforms.

3. Use according to claim 2, wherein the one or more further transglutaminase isoforms is selected from transglutaminase 1, transglutaminase 2, transglutaminase 3, transglutaminase 4, transglutaminase 5, transglutaminase 7, coagulation factor XIII or erythrocyte membrane protein band 4.2.

## Patentansprüche

1. Verwendung von Transglutaminase 6, oder einem antigen aktiven Fragment davon, zur Detektion von Autoantikörpern, die mit Transglutaminase 6 reagieren, zur *in vitro* Diagnose einer Autoimmunkrankheit oder zur Überwachung der Wirksamkeit der Therapie einer Autoimmunerkrankung, wobei die Autoimmunerkrankung Zöliakie, Stiff-Man-Syndrom, glutensensitive cerebelläre Ataxie, glutensensitive periphere Neuropathie, enteropathische Gluten-Ataxie, Kopfschmerz mit einer Anomalie der weißen Substanz, glutensensitive Myopathie oder myoklonische glutensensitive Ataxie ist.

2. Verwendung gemäß Anspruch 1, wobei die Detektion der Transglutaminase 6-Autoantikörper mit der Detektion von Autoantikörpern gegen eine oder mehrere weitere Transglutaminase-Isoformen kombiniert wird.

3. Verwendung gemäß Anspruch 2, wobei die eine oder mehrere weitere Transglutaminase-Isoformen aus Transglutaminase 1, Transglutaminase 2, Transglutaminase 3, Transglutaminase 4, Transglutaminase 5, Transglutaminase 7, Gerinnungsfaktor XIII oder Erythrozytenmembran-Proteinbande 4.2 ausgewählt werden.

## Revendications

1. Utilisation de la transglutaminase 6, ou d'un fragment antigéniquement actif d'elle, pour la détection d'autoanticorps réagissant avec la transglutaminase 6 pour diagnostic in vitro d'une maladie auto-immune ou pour le contrôle de l'efficacité de la thérapie d'une maladie auto-immune, dans laquelle la maladie auto-immune est coeliaquie, syndrome de l'homme raide, ataxie cérébelleuse sensible au gluten, neuropathie périphérique sensible au gluten, ataxie au gluten entéropathique, mal de tête avec une anomalie de la substance blanche, myopathie sensible au gluten ou ataxie myoclonique sensible au gluten.

2. Utilisation selon la revendication 1, dans laquelle la détection des anticorps de la transglutaminase 6 est combinée avec la détection d'anticorps contre une autre ou plus d'isoformes de la transglutaminase.

3. Utilisation selon la revendication 2, dans laquelle l'une autre ou plus d'isoformes de la transglutaminase sont choisies parmi transglutaminase 1, transglutaminase 2, transglutaminase 3, transglutaminase 4, transglutaminase 5, transglutaminase 7, facteur XIII de coagulation ou bande de protéine de membrane de l'érythrocyte 4.2.
